**Europäisches Patentamt**

**European Patent Office**

**Office européen des brevets**

(19)

(11) Veröffentlichungsnummer: **0 066 258**
**B1**

(12) **EUROPÄISCHE PATENTSCHRIFT**

(45) Veröffentlichungstag der Patentschrift:
**29.01.86**

(21) Anmeldenummer: **82104628.1**

(22) Anmeldetag: **27.05.82**

(51) Int. Cl.⁴: **C 07 C 9/04,** C 07 C 1/02,
F 22 G 1/00

(54) Verfahren zur Erzeugung von überhitztem Dampf im Wärmeaustausch mit einem katalytisch zu methanisierenden, Kohlenmonoxid, Kohlendioxid und Wasserstoff enthaltenden Synthesegas sowie Vorrichtung zur Durchführung des Verfahrens.

(30) Priorität: **03.06.81 DE 3121991**

(43) Veröffentlichungstag der Anmeldung:
**08.12.82 Patentblatt 82/49**

(45) Bekanntmachung des Hinweises auf die Patenterteilung:
**29.01.86 Patentblatt 86/5**

(84) Benannte Vertragsstaaten:
**FR GB IT NL SE**

(56) Entgegenhaltungen:
**DE - A - 2 705 141**
**DE - A - 2 949 588**

(73) Patentinhaber: **Kernforschungsanlage Jülich Gesellschaft mit beschränkter Haftung, Postfach 1913, D-5170 Jülich 1 (DE)**
Patentinhaber: **Rheinische Braunkohlenwerke AG., Stüttgenweg 2, D-5000 Köln 41 (DE)**

(72) Erfinder: **Höhlein, Bernd, Dr., Am Vogeldriesch 1b, D-5172 Linnich-Tetz (DE)**
Erfinder: **Vorwerk, Manfred, Aachener Strasse 112, D-4150 Erkelenz (DE)**
Erfinder: **Boltendahl, Udo, Dr., Klappholz-Westscheide, D-2381 Havetoft (DE)**

EP 0 066 258 B1

## Beschreibung

Die Erfindung bezieht sich auf ein Verfahren zur Erzeugung von überhitztem Dampf im Wärmeaustausch mit einem katalytisch zu methanisierenden, Kohlenmonoxid, Kohlendioxid und Wasserstoff enthaltenden Synthesegas, von dem ein Teilstrom zunächst zumindest einen ersten innengekühlten Reaktor durchströmt, in dem Sattdampf gebildet wird, und anschliessend vereint mit dem übrigen Synthesegas hintereinander zumindest einem adiabaten Reaktor mit nachgeschaltetem Wärmetauscher, in dem der Sattdampf überhitzt wird, und einem zweiten innengekühlten Reaktor zugeführt wird, dem als Kühlmittel Frischwasser zuströmt. Gegenstand der Erfindung ist auch eine Vorrichtung zur Durchführung des Verfahrens.

Die Umwandlung eines Kohlenmonoxid, Kohlendioxid und Wasserstoff enthaltenden Synthesegases ist, da sie exotherm verläuft, zur Gewinnung von Energie nutzbar. Synthesegase dieser Art lassen sich unter Wärmezufuhr (insbesondere unter Ausnutzung von Kernenergie) durch Spaltung eines Kohlenwasserstoffe enthaltenden Gases erzeugen, wobei zumindest ein Teil des Spaltgases als Energieträger einem Verbraucher zugeführt wird, vergleiche hierzu DE-AS 1 601 001. Beim Verbraucher erfolgt die Umwandlung des Synthesegases, wobei Nutzwärme ausgekoppelt wird. Zur Spaltung eignen sich vor allem Methan und höherwertige Kohlenwasserstoffe, wie Äthan, Propan, Butan.

Es ist bekannt, die Umwandlung der Synthesegase in Katalysator enthaltenden adiabaten und/ oder innengekühlten Reaktoren durchzuführen. Bei adiabaten Reaktoren erfolgt die Wärmeauskopplung in nachgeschalteten Wärmetauschern, bei innengekühlten Reaktoren wird die Wärme unmittelbar dem katalytischen Prozess entzogen. Zur Dampferzeugung weisen die innengekühlten Reaktoren wasserdurchströmte Kühlsysteme auf, die innerhalb des Katalysatorbettes angeordnet sind. Bei Einsatz adiabater Reaktoren ist eine für den Katalysator maximal zulässige Betriebstemperatur zu beachten, die nicht überschritten werden darf, soll das Katalysatormaterial stabil bleiben. Bekannt ist es, die Betriebstemperatur durch Rückführen eines Teils des dem adiabatischen Reaktor entströmenden Gases und Mischen des Gases mit dem Synthesegas oder durch Wasserdampfzufuhr zum Synthesegas zu steuern. Während bei der Rückführung von Gas zusätzliche Kreislaufkomponenten, insbesondere Kompressoren erforderlich sind, wird bei Wasserdampfzufuhr die Wirtschaftlichkeit reduziert.

Bei einer Prozessführung mit innengekühlten Reaktoren ist vor allem dem Wärmedurchgang zwischen Katalysatorbett und Kühlmittel Rechnung zu tragen. Vorteil des innengekühlten Reaktors ist die Möglichkeit, eine weitgehende Methanisierung durch entsprechende Abkühlung des Gases im Reaktor zu erreichen, ohne den Katalysator bei unzulässig hohen Temperaturen zu belasten.

Bekannt ist, Synthesegas in Anlagen zu methanisieren, die innengekühlte Reaktoren neben adiabaten Reaktoren enthalten, vgl. hierzu B. Höhlein, «Methanisierungsverfahren unter besonderer Berücksichtigung der Arbeiten zum NFE-Projekt», Berichte der Kernforschungsanlage Jülich, JÜL-1589, Mai 1979. Solche Anlagen erlauben eine Führung des Prozesses ohne Rückleiten von Gas mittels störanfälligen Kompressoren. Aus DE-A-2 705 141 und DE-A-2 949 588 ist es zur Erzeugung überhitzten Sattdampfes bekannt, Synthesegas durch einen adiabaten Reaktor, dem ein Wärmetauscher nachgeschaltet ist, und einen innengekühlten Reaktor hindurchzuleiten. Dabei wird der Sattdampf in den innengekühlten Reaktoren bei wasserseitig konstanter Temperatur, nämlich Verdampfungstemperatur, erzeugt und im Wärmetauscher überhitzt. Eine hohe Produktgasqualität bei gleichzeitiger Ausnutzung der gesamten die Reaktoren durchströmenden Kühlmittelmenge zur Erzeugung von thermisch hochwertigem Sattdampf wird nicht erreicht.

Aufgabe der Erfindung ist es, die Methanisierung von Synthesegas wirtschaftlicher zu gestalten und unter weitgehender Ausnutzung der bei der katalytischen Umwandlung auskoppelbaren Wärmemengen eine Optimierung der Dampferzeugung im Prozess zu erreichen.

Diese Aufgabe wird bei einem Verfahren der eingangs erwähnten Art gemäss der Erfindung durch die im Patentanspruch 1 angegebenen Massnahmen gelöst. Das in Sattdampf zu überführende Frischwasser)+ durchströmt die Kühlsysteme der beiden innengekühlten Reaktoren nacheinander, wobei das Frischwasser im zweiten innengekühlten Reaktor auf annähernd Sattdampftemperatur erwärmt wird und der nur von einem Teil des Synthesegases durchströmte erste innengekühlte Reaktor im wesentlichen die Verdampfungswärme zur Sattdampfbildung liefert.

Ein Massenausgleich zwischen den Kühlsystemen der innengekühlten Reaktoren wird in weiterer Ausgestaltung der Erfindung durch die im Patentanspruch 2 angegebenen Massnahmen unterstützt. Danach strömt das Frischwasser vom zweiten innengekühlten Reaktor zum ersten innengekühlten Reaktor über eine Dampftrommel, in die auch der im ersten innengekühlten Reaktor erzeugte Sattdampf eingeleitet wird. Der gebildete Sattdampf strömt dann aus dem Dampfraum der Dampftrommel zum Wärmetauscher, in dem er überhitzt wird. Die Dampftrommel dient einer gleichmässigen Prozessführung. Die im Produktgas, das dem letzten innengekühlten Reaktor entströmt, noch enthaltene Wärme wird durch die in Patentanspruch 3 und 4 gekennzeichneten Massnahmen in Wärmetauschern, die dem innengekühlten Reaktor nachgeschaltet sind, zur Vorerhitzung von Frischwasser und Synthesegas genutzt. Eine weitere Massnahme zur Aufheizung des Synthesegases ist Patentanspruch 5 entnehmbar.

---

)+ Der Begriff Frischwasser umfasst im wesentlichen in üblicher Weise aufbereitetes Kesselspeisewasser.

Eine Vorrichtung zur Durchführung des Verfahrens gemäss der Erfindung ist im Patentanspruch 6 angegeben. Die Vorrichtung weist in Strömungsrichtung des Gases gesehen, hintereinander geschaltet zumindest einen ersten innengekühlten Reaktor, einen adiabaten Reaktor mit nachgeschaltetem Wärmetauscher und einen zweiten innengekühlten Reaktor auf. Der erste innengekühlte Reaktor wird nur von einem Teil des Synthesegases durchströmt, das danach vereint mit dem übrigen Synthesegas dem adiabaten Reaktor zugeführt wird. Die Kühlsysteme der beiden innengekühlten Reaktoren sind in der Weise hintereinander geschaltet, dass im zweiten innengekühlten Reaktor das Frischwasser erwärmt und im ersten innengekühlten Reaktor Sattdampf erzeugt wird. In weiterer Ausgestaltung der Erfindung nach Patentanspruch 7 ist zwischen den beiden Kühlsystemen der innengekühlten Reaktoren eine Dampftrommel vorgesehen, in der im zweiten innengekühlten Reaktor auf annähernd Sattdampftemperatur erhitztes Frischwasser eingeleitet wird. An der Dampftrommel sind auch Eingang und Ausgang des Kühlsystems des ersten innengekühlten Reaktors angeschlossen, in dem Heisswasser, das der Dampftrommel entnommen wird, in Sattdampf überführt wird. Aus dem Dampfraum der Dampftrommel strömt der Sattdampf zum Wärmetauscher, der dem adiabaten Reaktor nachgeschaltet ist, und wird dort überhitzt.

In weiterer Ausgestaltung der Erfindung wird die Wärmemenge des dem letzten innengekühlten Reaktor entströmenden Produktgases in weiteren vom Produktgas durchströmten Wärmetauschern zur Vorerhitzung von Frischwasser und Synthesegas genutzt, Patentansprüche 7 und 8. In einem weiteren Wärmetauscher, der dem der Sattdampfüberhitzung dienenden Wärmetauscher nachgeschaltet ist, wird das Synthesegas vor Einströmen in den ersten innengekühlten Reaktor weiter vorerhitzt.

Die Erfindung wird anhand eines Ausführungsbeispieles, das in der Zeichnung schematisch dargestellt ist, näher erläutert. Es zeigen im einzelnen:

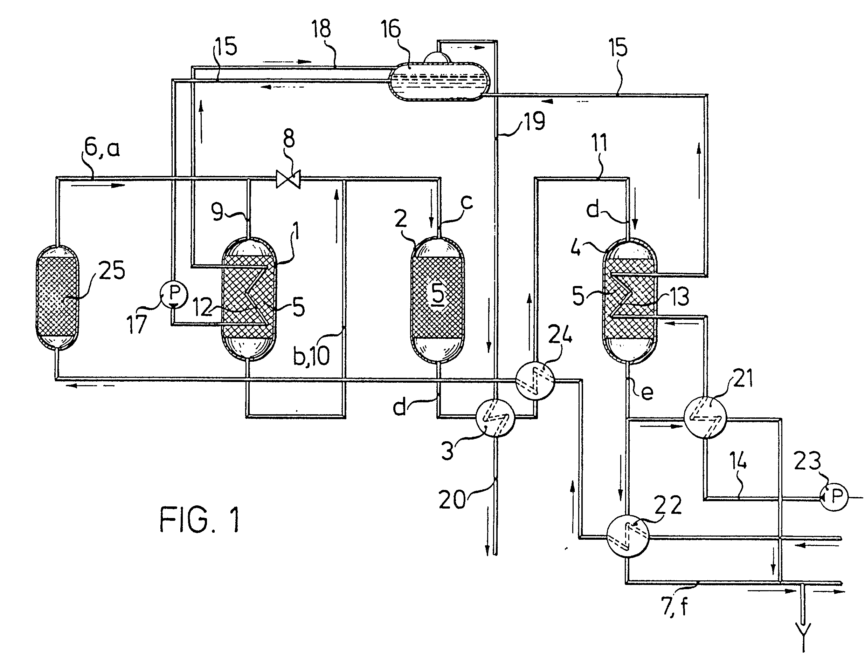

Figur 1: Anlage mit zwei innengekühlten und einem adiabaten Reaktor

Figur 2: tabellarische Übersicht über die Zusammensetzung des Gases an verschiedenen Stellen der Anlage.

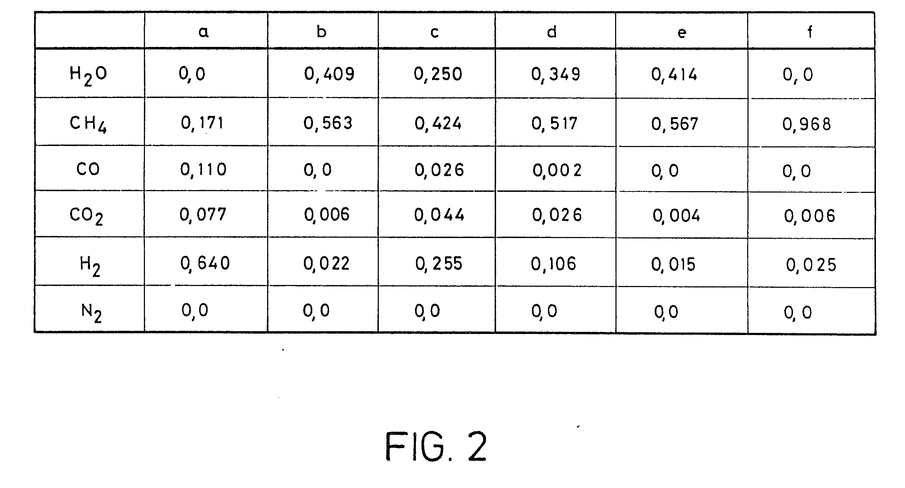

Wie aus der Zeichnung ersichtlich ist, weist die Anlage einen ersten innengekühlten Reaktor 1, einen adiabaten Reaktor 2, dem ein Wärmetauscher 3 nachgeschaltet ist, und einen zweiten innengekühlten Reaktor 4 auf. Alle Reaktoren sind mit Katalysator gefüllt, über den Gas zur Umwandlung leitbar ist. Als Synthesegas wird ein zumindest 50 Vol.% Wasserstoff und 10 Vol.% Kohlenmonoxid und Kohlendioxid enthaltendes Gas, als Katalysator ein nickelhaltiges Katalysatorbett 5 genutzt. Im Ausführungsbeispiel strömt den Reaktoren über eine Synthesegasleitung 6 ein etwa 17 Vol.% $CH_4$, 11 Vol.% CO, 8 Vol.% $CO_2$, 64 Vol.% $H_2$, Restvolumenprozente Stickstoff und gegebenenfalls geringe Mengen höherwertige Kohlenwasserstoffe enthaltendes Synthesegas zu (Fig. 2, Tabellenwert a), das in Gegenwart des Katalysators in den Reaktoren zu einem methanreichen Produktgas mit etwa 97 Vol.% $CH_4$ und Restvolumenprozente Kohlendioxid, Wasserstoff und Stickstoff (Fig. 2, Tabellenwert f) umgewandelt und über eine Produktgasleitung 7 aus der Anlage abgeführt wird.

Der erste der innengekühlten Reaktoren, der Reaktor 1, ist an der einen Durchflussregler 8 aufweisenden Synthesegasleitung 6 mittels einer Zweigleitung 9 angeschlossen, und wird nur von einem Teil des Synthesegases durchströmt, der vom Ausgang des Reaktors 1 über eine Zusammenführungsleitung 10 vor Eintritt in den adiabaten Reaktor 2 mit dem übrigen Teil des Synthesegases wieder vereint wird. Vom adiabaten Reaktor 2 wird das Gas mittels einer Verbindungsleitung 11 über den Wärmetauscher 3 zum zweiten innengekühlten Reaktor 4 geführt.

In beiden innengekühlten Reaktoren 1 und 4 sind jeweils innerhalb der Katalysatorbetten Kühlsysteme 12, 13 angeordnet. Am Kühlsystem 13 des zweiten innengekühlten Reaktors 4 ist ein Frischwasserzulauf 14 angeschlossen. Vom Ausgang des Kühlsystems 13 führt eine Heisswasserleitung 15 zum Eingang des Kühlsystems 12 des ersten innengekühlten Reaktors 1. Mit der Heisswasserleitung 15 ist eine Dampftrommel 16 verbunden, aus der das Heisswasser mittels einer Pumpe 17 zum Kühlsystem 12 des ersten innengekühlten Reaktors 1 gefördert wird. An der Dampftrommel 16 münden eine am Ausgang des Kühlsystems 12 des ersten innengekühlten Reaktors 1 angeschlossene Sattdampfleitung 18 und eine Sattdampfzuführung 19, in der der Sattdampf zur Überhitzung zum Wärmetauscher 3 geführt wird. Vom Ausgang des Wärmetauschers 3 strömt der überhitzte Wasserdampf dann über eine Dampfleitung 20 beispielsweise Dampfturbinen mit Generatoren zur Erzeugung elektrischer Energie zu. Die zuletzt genannten Aggregate sind in der Zeichnung nicht wiedergegeben.

Das dem Kühlsystem 13 des zweiten innengekühlten Reaktors 4 zugeführte Frischwasser wird im Reaktor 4 auf Sattdampftemperatur erwärmt. Im Kühlsystem 12 des ersten innengekühlten Reaktors erfolgt dann die Überführung des mittels der Pumpe 17 der Dampftrommel 16 entnommenen Heisswassers in Sattdampf. Die den ersten innengekühlten Reaktor 1 durchströmende Teilgasmenge des Synthesegases ist dabei so eingestellt, dass das Gas nach Zusammenführen der beiden Teilgasmengen am Eingang des adiabaten Reaktors 2 eine Temperatur im Temperaturbereich zwischen 250 und 350°C aufweist, und dass am Ausgang des adiabaten Reaktors eine Temperatur zwischen 600° und 700°C erreicht wird. Im Ausführungsbeispiel beträgt die Eintrittstemperatur in den ersten innengekühlten Reaktor tor 330°C bei einem Druck von 40 bar, die Eingangstemperatur am adiabaten Reaktor 325°C bei 39 bar und die Ausgangstemperatur 675°C bei 38,5 bar. Mit der zuletzt genannten Temperatur tritt das Gas in den Wärmetauscher 3 ein und überhitzt den von der Dampftrommel 16 abströmenden Sattdampf. Der zweite innengekühlte Reaktor 4 ist auf eine Eintrittstemperatur zwischen 250 und 350°C eingestellt, im Ausführungsbeispiel auf eine Temperatur von 300°C bei einem mittleren Druck von 37,5 bar. Das Gas wird in diesem letzten innengekühlten Reaktor in ein methanreiches Produktgas umgewandelt.

Die Zusammensetzung des Gases beim Durchlauf der Anlage ist aus Figur 2 ersichtlich. Unter a) ist in der Tabelle – wie oben bereits erläutert – der Eingangszustand des Synthesegases angegeben. Dem Tabellenwert b) ist die Zusammensetzung der dem ersten innengekühlten Reaktor 1 zugeführten Teilmenge des Gases nach Durchströmen des Reaktors 1 an dessen Ausgang entnehmbar. Die Teilmenge weist an dieser Stelle etwa die folgende Zusammensetzung auf: 41 Vol.% $H_2O$, 56 Vol.% $CH_4$, 0,6 Vol.% $CO_2$, 2 Vol.% $H_2$, Rest Stickstoff. Nach Zusammenführen mit dem übrigen Teil des Synthesegases stellt sich am Eingang des adiabaten Reaktors 2 etwa eine Zusammensetzung von 25 Vol.% $H_2O$, 42 Vol.% $CH_4$, 3 Vol.% CO, 4 Vol.% $CO_2$, 26 Vol.% $H_2$ Rest Stickstoff ein (Tabellenwert c) und am Ausgang des adiabaten Reaktors 2 wird nach teilweiser Methanisierung etwa eine Zusammensetzung von 35 Vol.% $H_2O$, 52 Vol.% $CH_4$, 0,2 Vol.% CO, 3 Vol.% $CO_2$, 11 Vol.% $H_2$, Rest Stickstoff erreicht (Tabellenwert d). Nach Durchströmen des zweiten innengekühlten Reaktors 4 sind im Produktgas etwa 41 Vol.% $H_2O$, 57 Vol.% $CH_4$, 0,4 Vol.% $CO_2$ und 1 Vol.% $H_2$, Rest Stickstoff enthalten (Tabellenwert e) und nach weiterer Abkühlung des Produktgases auf 40°C wird bei einem Druck von 36,5 bar schliesslich ein Produktgas der in der Tabelle unter f) angegebenen Zusammensetzung erreicht.

Um die Restwärme des dem zweiten innengekühlten Reaktor mit 300°C abströmenden Produktgases auszunutzen, sind in der Synthesegasanlage am Ausgang des zweiten innengekühlten Reaktors 4 noch zwei vom Produktgas durchströmbare Wärmetauscher 21, 22 angeschlossen. Einer dieser Wärmetauscher, der Wärmetauscher 21, dient der Vorwärmung von Frischwasser. Im Ausführungsbeispiel wird das mittels einer Wasserpumpe 23 bei 50°C und 3 bar geförderte und auf einen Druck von 110 bar gebrachte Frischwasser auf 160°C vorgewärmt. Im Kühlsystem 12 des zweiten innengekühlten Reaktors erreicht das Frischwasser dann bei gleichem Druck die Sattdampftemperatur von 318°C.

Im Wärmetauscher 22 wird dem ersten innengekühlten Reaktor 1 zuströmendes Synthesegas vorerhitzt. Das Synthesegas strömt der Anlage mit 10°C bei einem Druck von 41 bar zu, und wird im Wärmetauscher 22 bis auf 230°C erwärmt. In einem weiteren Wärmetauscher 24, der in Strömungsrichtung des Gases gesehen dem der Überhitzung des Sattdampfes dienenden Wärmetauscher 3 nachgeschaltet ist, erfolgt eine weitere Aufheizung des Synthesegases bis auf 340°C. Das Synthesegas durchströmt bei dieser Temperatur zur Entschwefelung noch einen Gasreiniger 25, der mit Zinkoxid (ZnO) gefüllt ist, bevor schliesslich ein Teilstrom des Synthesegases dem ersten innengekühlten Reaktor 1 und die gesamte Gasmenge dem adiabaten Reaktor 2 zugeführt wird.

Die Anlage weist innerhalb des Gasstroms keine Förderaggregate auf. Die Prozessführung mit zwei innengekühlten Reaktoren und einem adiabaten Reaktor ist so gestaltet, dass die zulässige Katalysatortemperatur im adiabaten Reaktor nicht überschritten wird. Es ist dafür Sorge getragen, dass das gesamte mit einer Temperatur zwischen 40°C und 50°C die Anlage erreichende Frischwasser durch die bei der Umwandlung des Synthesegases in den Reaktoren freiwerdende und im Produktgas enthaltene Wärmemenge zunächst auf Sattdampftemperatur erhitzt, dann in Sattdampf und abschliessend in überhitzten Dampf umgeformt wird. Dieses Ziel kann mit dem beschriebenen Verfahren erreicht werden

  – ohne Wasserdampfzugabe ins Synthesegas

  – ohne Produktgasrückführung mittels Kompressoren und

  – ohne Nutzung einer externen Wärmezufuhr zur Vorwärmung von Frischwasser und Synthesegas.

## Patentansprüche

1. Verfahren zur Erzeugung von überhitztem Dampf im Wärmeaustausch mit einem katalytisch zu methanisierenden, Kohlenmonoxid, Kohlendioxid und Wasserstoff enthaltenden Synthesegas, von dem ein Teilstrom zunächst zumindest einen ersten innengekühlten Reaktor durchströmt, in dem Sattdampf gebildet wird, und anschliessend vereint mit dem übrigen Synthesegas hintereinander zumindest einem adiabaten Reaktor mit nachgeschaltetem Wärmetauscher, in dem der Sattdampf überhitzt wird und einem zweiten innengekühlten Reaktor zugeführt wird, dem als Kühlmittel Frischwasser zuströmt, dadurch gekennzeichnet, dass das Frischwasser im zweiten innengekühlten Reaktor auf annähernd Sattdampftemperatur vorgewärmt und in den ersten innengekühlten Reaktor überführt wird.

2. Verfahren nach Patentanspruch 1, dadurch gekennzeichnet, dass das vorerhitzte Frischwasser aus dem zweiten innengekühlten Reaktor über eine Dampftrommel zum ersten innengekühlten Reaktor strömt und dass der im ersten innengekühlten Reaktor erzeugte Sattdampf über die gleiche Dampftrommel zum Wärmetauscher zur Dampfüberhitzung geführt wird.

3. Verfahren nach Patentanspruch 1 oder 2, dadurch gekennzeichnet, dass das Frischwasser vor Eintritt in den zweiten innengekühlten Reaktor von aus diesem Reaktor abströmendem Produktgas vorgewärmt wird.

4. Verfahren nach Patentanspruch 3, dadurch gekennzeichnet, dass das Frischwasser von einem Teilstrom des aus dem zweiten innengekühlten Reaktor abströmenden Produktgases vorgewärmt wird und der übrige Teil des Produktgases der Vorerhitzung von Synthesegas vor dessen Eintritt in den ersten innengekühlten Reaktor dient.

5. Verfahren nach einem der vorhergehenden Ansprüche, dadurch gekennzeichnet, dass dem ersten innengekühlten Reaktor zugeführtes Synthesegas von aus dem adiabaten Reaktor abströmendem Gas vorgewärmt wird.

6. Vorrichtung zur Durchführung des Verfahrens nach Anspruch 1 mit Reaktoren, die zur Methanisierung eines Kohlenmonoxid, Kohlendioxid und Wasserstoff enthaltenden Synthesegases mit dem Gas kontaktierbaren Katalysator enthalten und hintereinander geschaltet sind, wobei in Strömungsrichtung des Gases gesehen zumindest ein von einem Teil des Synthesegases durchströmbarer erster innengekühlter Reaktor mit einem wasserdurchströmten Kühlsystem angeordnet ist, dessen Ausgang an eine Wasser/Sattdampfleitung angeschlossen ist, und zumindest ein vom Gas durchströmter, adiabater Reaktor, ein Wärmetauscher zur Überhitzung des Sattdampfes und ein zweiter innengekühlter Reaktor mit wasserdurchströmtem Kühlsystem nachgeschaltet sind, der einen Frischwasserzulauf aufweist, dadurch gekennzeichnet, dass vom Ausgang des Kühlsystems (13) des zweiten innengekühlten Reaktors (4) eine vorerhitztes Frischwasser führende Heisswasserleitung (15) zum Eingang des Kühlsystems (12) des ersten innengekühlten Reaktors (1) geführt ist.

7. Vorrichtung nach Anspruch 6, dadurch gekennzeichnet, dass die Heisswasserleitung (15) über eine Dampftrommel (16) geführt ist, in die die am Ausgang des Kühlers (12) des ersten innengekühlten Reaktors (1) angeschlossene Wasser/Dampfleitung (18) mündet.

8. Vorrichtung nach Anspruch 6 oder 7, dadurch gekennzeichnet, dass dem zweiten innengekühlten Reaktor (4) ein vom Produktgas durchströmbarer Wärmetauscher (21) zur Vorwärmung von Frischwasser nachgeschaltet ist.

9. Vorrichtung nach Anspruch 6, 7, oder 8, dadurch gekennzeichnet, dass dem zweiten innengekühlten Reaktor (4) parallel zum Frischwasser erhitzenden Wärmetauscher (21) ein von einem Teil des Produktgases durchströmbarer Wärmetauscher (22) zur Vorerhitzung des dem ersten innengekühlten Reaktor (1) zugeführten Synthesegases nachgeschaltet ist.

10. Vorrichtung nach Anspruch 9, dadurch gekennzeichnet, dass dem Wärmetauscher (22) zur Vorerhitzung von Synthesegas in Strömungsrichtung des Synthesegases gesehen ein weiterer Wärmetauscher (24) zur Vorerhitzung des Synthesegases nachgeschaltet ist, der in eine vom adiabaten Reaktor (2) abströmendes Gas führende Verbindungsleitung (11) zwischen Wärmetauscher (3) und zweitem innengekühlten Reaktor (4) eingesetzt ist.

## Claims

1. Process for the preparation of superheated steam in heat exchange with a synthesis gas which is to be methanised catalytically and which comprises carbon monoxide, carbon dioxide and hydrogen, and whereof a part flow flows in the first instance through at least one first internally cooled reactor in which saturated steam is formed and then, united with the remainder of the synthesis gas, is fed in succession to at least one adiabatic reactor with downstream heat exchanger in which the saturated steam is superheated, and to a second internally cooled reactor into which fresh water flows as coolant, characterised in that the fresh water is pre-heated in the second internally cooled reactor to approximately saturated steam temperature and transferred into the first internally cooled reactor.

2. Process according to claim 1, characterised in that the preheated fresh water flows from the second internally cooled reactor by way of a steam drum to the first internally cooled reactor, and that the saturated steam produced in the first internally cooled reactor is conducted via the same steam drum to the heat exchanger for steam superheating.

3. Process according to claim 1 or 2, characterised in that the fresh water, before entry into the second internally cooled reactor, is preheated by product gas flowing out of this reactor.

4. Process according to claim 3, characterised in that the fresh water is preheated by a part-flow of the product gas flowing out of the second internally cooled reactor, and the remainder of the product gas serves to preheat synthesis gas before the entry thereof into the first internally cooled reactor.

5. Process according to one of the preceding claims, characterised in that synthesis gas fed to the first internally cooled reactor is preheated by gas flowing out of the adiabatic reactor.

6. Apparatus for performing the process according to claim 1, with reactors which contain a catalyst adapted to be brought into contact with the gas for methanisation of a synthesis gas containing carbon monoxide, carbon dioxide and hydrogen, and which are connected in series to one another, and, as viewed in the direction of flow of the gas, there is arranged at least one first internally cooled reactor, which can have part of the synthesis gas flowing through and which has a cooling system with water throughflow, whose output is connected to a water/saturated steam conduit, followed by at least one adiabatic reactor through which the gas flows, a heat exchanger for superheating the saturated steam, and a second internally cooled reactor with a water-throughflow cooling system and also with a fresh water inflow, characterised in that from the output of the cooling system (13) of the second internally cooled reactor (4) a hot-water conduit (15) conducting preheated fresh water is taken to the inlet of the cooling system (12) of the first internally cooled reactor (1).

7. Apparatus according to claim 6, characterised in that the hot-water conduit (15) extends by way of a steam drum (16) into which opens the water/steam conduit (18) connected to the output of the cooling device (12) of the first internally cooled reactor (1).

8. Apparatus according to claim 6 or 7, characterised in that downstream of the second internally cooled reactor (4) there is connected a heat exchanger (21), adapted to have the product gas flowing through, for the preheating of fresh water.

9. Apparatus according to claim 6, 7 or 8, characterised in that there is connected to the downstream side of the second internally cooled reactor (4), in parallel with the heat exchanger (21) heating fresh water, a heat exchanger (22) through which part of the product gas can flow, for preheating of the synthesis gas fed to the first internally cooled reactor (1).

10. Apparatus according to claim 9, characterised in that there is connected to the downstream side of the heat exchanger (22) for the preheating of synthesis gas, as viewed in the direction of flow of the synthesis gas, a further heat exchanger (24) for the preheating of the synthesis gas, this further exchanger being situated in a connecting conduit (11) which conducts gas flowing out of the adiabatic reactor (2) and is arranged between heat exchanger (3) and second internally cooled reactor (4).

## Revendications

1. Procédé de production de vapeur d'eau surchauffée par échange de chaleur avec un gaz de synthèse, à méthaniser catalytiquement, qui contient du monoxyde de carbone, du dioxyde de carbone et de l'hydrogène, dont un courant partiel passe d'abord au moins dans un premier réacteur refroidi intérieurement dans lequel se forme de la vapeur saturée et, ensuite, réuni au reste du gaz de synthèse, est envoyé successivement au moins à un réacteur adiapatique à échangeur de chaleur en aval dans lequel la vapeur saturée est surchauffée, et à un deuxième réacteur refroidi intérieurement, auquel est envoyée de l'eau fraîche comme agent réfrigérant, caractérisé en ce qu'il consiste à préchauffer l'eau fraîche dans le deuxième réacteur refroidi intérieurement, à peu près à la température de la vapeur saturée, et à la transvaser dans le premier réacteur refroidi intérieurement.

2. Procédé suivant la revendication 1, caractérisé en ce qu'il consiste à faire s'écouler l'eau fraîche préchauffée du deuxième réacteur refroidi intérieurement au premier réacteur refroidi intérieurement au premier réacteur refroidi intérieurement en la faisant passer par un ballon de vapeur, et à envoyer la vapeur saturée produite dans le premier réacteur refroidi intérieurement à l'échangeur de chaleur destiné à surchauffer la vapeur en la faisant passer par le même ballon de vapeur.

3. Procédé suivant la revendication 1 ou 2, caractérisé en ce qu'il consiste à préchauffer l'eau fraîche avant l'entrée dans le deuxième réacteur refroidi intérieurement par le gaz produit sortant de ce réacteur.

4. Procédé suivant la revendication 3, caractérisé en ce qu'il consiste à préchauffer l'eau fraîche par un courant partiel du gaz produit sortant du deuxième réacteur refroidi intérieurement, et à se servir de la partie restante du gaz produit pour préchauffer du gaz de synthèse avant son entrée dans le premier réacteur refroidi intérieurement.

5. Procédé suivant l'une des revendications précédentes, caractérisé en ce qu'il consiste à préchauffer le gaz de synthèse envoyé au premier réacteur refroidi intérieurement, par le gaz sortant du réacteur adiabatique.

6. Installation pour la mise en œuvre du procédé suivant la revendication 1, comprenant des réacteurs qui, pour la méthanisation d'un gaz de synthèse contenant du monoxyde de carbone, du dioxyde de carbone et de l'hydrogène, contiennent un catalyseur pouvant être mis en contact avec le gaz et sont montés en série, au moins un premier réacteur, considéré suivant le sens d'écoulement du gaz, refroidi intérieurement pouvant être parcouru par une partie du gaz de synthèse, étant prévu avec un système de refroidissement parcouru par de l'eau et dont la sortie est raccordée à un conduit eau/vapeur saturée et au moins un réacteur adiabatique parcouru par du gaz, un échangeur de chaleur pour surchauffer la vapeur saturée et un deuxième réacteur refroidi intérieurement, à système de refroidissement parcouru par de l'eau, étant montés en aval, ce système de refroidissement présentant une arrivée d'eau fraîche, caractérisée en ce qu'un conduit d'eau chaude (15), conduisant de l'eau fraîche surchauffée, va de la sortie du système de refroidissement (13) du second réacteur refroidi intérieurement (4) à l'entrée du système de refroidissement (12) du premier réacteur refroidi intérieurement (1).

7. Installation suivant la revendication 6, caractérisée en ce que le conduit d'eau chaude (15) passe par un ballon de vapeur (16), dans lequel débouche le conduit pour de l'eau/vapeur (18) raccordé à la sortie du dispositif de refroidissement (12) du premier réacteur refroidi intérieurement (1).

8. Installation suivant la revendication 6 ou 7, caractérisé en ce qu'en aval du second réacteur refroidi intérieurement (4) est monté un échangeur de chaleur (21) parcouru par du gaz produit et destiné à préchauffer de l'eau fraîche.

9. Installation suivant la revendication 6, 7 ou 8, caractérisée en ce qu'en aval du deuxième réacteur refroidi intérieurement (4), et en parallèle à l'échangeur de chaleur (21) réchauffant l'eau fraîche, est monté un échangeur de chaleur (22) pouvant être parcouru par une partie du gaz produit et destiné à préchauffer le gaz de synthèse envoyé au premier réacteur refroidi intérieurement (1).

10. Installation suivant la revendication 9, caractérisée en ce qu'en aval, considéré suivant le sens d'écoulement du gaz de synthèse, de l'échangeur de chaleur (22) destiné à préchauffer du gaz de synthèse, est monté un autre échangeur de chaleur (24) destiné à préchauffer du gaz de synthèse et placé dans un conduit de liaison (11) conduisant du gaz s'écoulant du réacteur adiabatique (2) et interposé entre l'échangeur de chaleur (3) et le deuxième réacteur refroidi intérieurement (4).

FIG. 1

0 066 258

| | a | b | c | d | e | f |
|---|---|---|---|---|---|---|
| $H_2O$ | 0,0 | 0,409 | 0,250 | 0,349 | 0,414 | 0,0 |
| $CH_4$ | 0,171 | 0,563 | 0,424 | 0,517 | 0,567 | 0,968 |
| $CO$ | 0,110 | 0,0 | 0,026 | 0,002 | 0,0 | 0,0 |
| $CO_2$ | 0,077 | 0,006 | 0,044 | 0,026 | 0,004 | 0,006 |
| $H_2$ | 0,640 | 0,022 | 0,255 | 0,106 | 0,015 | 0,025 |
| $N_2$ | 0,0 | 0,0 | 0,0 | 0,0 | 0,0 | 0,0 |

FIG. 2